# EUROPEAN PATENT SPECIFICATION

(11) **EP 3 233 115 B1**
(45) Date of publication and mention of the grant of the patent: **17.02.2021**
(21) Application number: 15871126.7
(22) Date of filing: 17.12.2015
(51) Int. Cl.: C12Q 1/6883

(54) **METHODS AND COMPOSITIONS RELATED TO TRANSPLANT-ASSOCIATED THROMBOTIC MICROANGIOPATHY**
VERFAHREN UND ZUSAMMENSETZUNGEN IM ZUSAMMENHANG MIT TRANSPLANTASSOZIIERTER THROMBOTISCHER MIKROANGIOPATHIE
PROCÉDÉS ET COMPOSITIONS RELATIFS À LA MICROANGIOPATHIE THROMBOTIQUE ASSOCIÉE À UN TRANSPLANT

(30) Priority: 19.12.2014 US 201462094802 P
(43) Date of publication of application: 25.10.2017
(73) Proprietor: Children's Hospital Medical Center, Cincinnati, Ohio 45229-3039 (US)
(72) Inventor: JODELE, Sonata, Montgomery, OH 45242 (US); ZHANG, Kejian, Cincinnati, OH 45241 (US); DAVIES, Stella, Cincinnati, OH 45230 (US)
(74) Representative: FRKelly
(86) International application number: PCT/US2015/066515
(87) International publication number: WO 2016/100745

(56) References cited:
- WO-A1-2015/039126
- US-A1- 2002 025 532
- US-A1- 2007 031 826
- US-A1- 2009 117 621
- US-A1- 2012 003 641
- M. NORIS ET AL: "Thrombotic Microangiopathy After Kidney Transplantation : Posttransplant TMA", AMERICAN JOURNAL OF TRANSPLANTATION, vol. 10, no. 7, 25 July 2010 (2010-07-25), pages 1517-1523, XP055460289, DK ISSN: 1600-6135, DOI: 10.1111/j.1600-6143.2010.03156.x
- T. BARBOUR ET AL: "Thrombotic microangiopathy and associated renal disorders", NEPHROLOGY DIALYSIS TRANSPLANTATION., vol. 27, no. 7, 1 July 2012 (2012-07-01), pages 2673-2685, XP055327896, GB ISSN: 0931-0509, DOI: 10.1093/ndt/gfs279
- ELENA ROMAN-ORTIZ ET AL: "Eculizumab long-term therapy for pediatric renal transplant in aHUS with CFH/CFHR1 hybrid gene", PEDIATRIC NEPHROLOGY., vol. 29, no. 1, 1 January 2014 (2014-01-01), pages 149-153, XP055309247, DE ISSN: 0931-041X, DOI: 10.1007/s00467-013-2591-8
- JODELE ET AL.: 'Abnormalities in the alternative pathway of complement in children with hematopoietic stem cell transplant-associated thrombotic microangiopathy' BLOOD vol. 122, no. 12, 19 September 2013, pages 2003 - 2007, XP055352965 DOI: 10.1182/BLOOD-2013-05-501445
- BARBOUR ET AL.: 'Thrombotic microangiopathy and associated renal disorders' NEPHROLOGY DIALYSIS TRANSPLANTATION vol. 27, no. 7, 01 July 2012, pages 2673 - 2685, XP055327896 DOI: 10.1093/NDT/GFS279

## Description

### FIELD OF THE INVENTION

The present invention relates to systems and methods for evaluating the risk of thrombotic microangiopathy, especially transplant-associated thrombotic microangiopathy. Some embodiments include selecting and administering a treatment for transplant-associated thrombotic microangiopathy in subjects identified as having elevated risk.

### BACKGROUND OF THE INVENTION

Transplant-associated thrombotic microangiopathy (TMA) is a complication associated with hematopoietic stem cell transplantation (HSCT). See Jodele, S., et al. (2014) Blood 124(4):645-653; Ricklin, D., et al. (2013) Blood 122(12):1997-1999; Meri, S. (2013) Eur. J. Intern. Med. 24(6):496-502; Chima, R.S. et al. (2013) Pediatr. Clin. North Am. 60(3):689-707; Abboud, I. et al. (2012) Semin. Hematol. 49(1):73-82. TMA can range from a mild, self-limited form to uncontrolled fulminant disease leading to death. The reasons behind the heterogeneity in disease severity are presently unknown.

TMA occurs when endothelial injury in the context of HSCT causes microangiopathic hemolytic anemia and platelet consumption, resulting in thrombosis and fibrin deposition in the microcirculation affecting multiple organs. Using rigorous prospective monitoring, TMA can be identified in 30-35% of HSCT recipients and progresses to life threatening disease in about half of those cases. In the absence of rigorous monitoring, severe TMA cases may not be properly diagnosed, and TMA-related deaths are often recorded as multi-system organ failure of unclear etiology. See Meri, S. (2013) Eur. J. Intern. Med. 24(6):496-502; Laskin, B.L., et al. (2011) Blood 118(6): 1452-1462, and Cho, B.S., et al. (2010) Transplantation 90(8):918-926.

At present, TMA can be diagnosed using published diagnostic criteria including, for example, those proposed by Cho, B.S. et al. (2010) Transplantation 90(8):918-926, including (1) lactate dehydrogenase (LDH) elevated above the upper limit of normal for the subject's age; (2) *de novo* thrombocytopenia with a platelet count <50 × 10⁹/L or a ≥50% decrease in the platelet count; (3) *de novo* anemia with a hemoglobin below the lower limit of normal or anemia requiring transfusion support; (4) microangiopathic changes defined as the presence of schistocytes in the peripheral blood or histologic evidence of microangiopathy on a tissue specimen; and (5) absence of a coagulopathy and a negative Coombs test, especially where the level of ADAMTS13 activity is measured in subjects with TMA to exclude a diagnosis of thrombotic thrombocytopenic purpura. Example of additional diagnostic criteria may include proteinuria, hypertension, and terminal complement activation.

Risk factors for poor outcome at TMA diagnosis are currently assessed using laboratory indicators and tests, including proteinuria >30 mg/dL and elevated sC5b-9 (the soluble membrane attack complex of the complement system); See Jodele et al. (2014) Blood 124(4): 645-53. However, there are currently no direct methods to address individual susceptibility to transplant associated TMA, and no available pre-transplant screening tools available to identify subjects at risk for severe TMA.

### SUMMARY OF THE INVENTION

Disclosed herein is a method for the prognosis of transplant associated thrombotic microangiopathy in a subject. In some embodiments, the method comprises the steps of (1) obtaining a biological sample containing genetic material from a subject; (2) determining genetic variants in complement-associated genes in the biological sample; and (3) assigning to the subject a risk level for the development of thrombotic microangiopathy, and for the severity of the thrombotic microangiopathy, based on the genetic variants determined in the biological sample, wherein the risk level corresponds to low risk where no genetic variants are identified, elevated risk where one genetic variant is identified, and high risk where two or more genetic variants are identified. In some embodiments, the biological sample is blood, saliva, skin, or other tissue or fluid.

Also disclosed herein is a method for treating transplant-associated thrombotic microangiopathy, comprising carrying out the prognostic method described above, and further directing observation of the subject for the appearance of symptoms of thrombotic microangiopathy in subjects in which one or more genetic markers are identified and directing the withdrawal of complement-activating therapies in such subjects. In some further embodiments, the method directs administering an effective amount of a prophylactic treatment or complement modifying therapy to a subject found to have more than one genetic variant. In some further embodiment, the complement modifying therapy is selected from the group consisting of Eculizumab, Rituximab, purified human C1 esterase inhibitor, or plasmapheresis.

Also disclosed herein is a kit for predicting the prognosis or risk of occurrence of thrombotic microangiopathy in a subject, comprising a set of nucleic acid primers configured to amplify or sequence complement-mediated genes from the subject. In some embodiments, the kit comprises a set of nucleic acid primers for amplification or sequencing of the *CD46, THBD, CFH, CFI CFB. C3, C5, CFD, CPHR1, CPHR3, CPHR4, CPHR5, CFP, C4BPA, CD59, CD55,* and *ADAMTS13* genes or for any subset thereof. In some embodiments, the kit further comprises probes configured to bind with genetic variants in loci from the group consisting of *CD46, THBD, CFH, CFI. CFB, C3, C5, CFD, CFHR1, CFHR3, CFHR4, CFHR5, CFP, C4BPA, CD59, CD55, and ADAMTS13,* or for any subset thereof. In some embodiments, the probes are labeled with a fluorescent dye, colorimetric dye, radioisotope, or spin-label. In some embodiments the probes may be attached to a solid support or presented in an array. In some embodiments, the primers for amplification of said loci incorporate a sequence tag. In some embodiments, the primers are attached to a solid support or are present in an array. In some further embodiments, the kit also comprises a sample collection device selected from the group consisting of a sample collection container, a sample collection tool, and a sample preservative. Nonetheless, the invention is defined in the appended claims.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

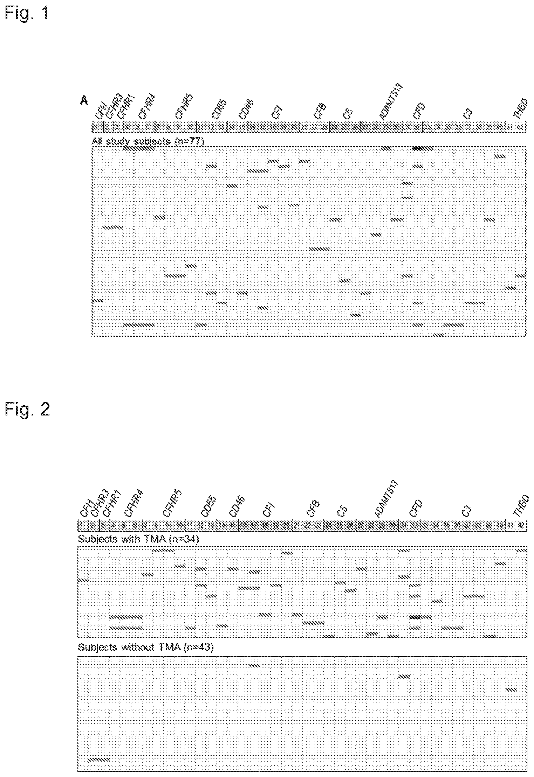

Figure 1 illustrates the distribution of a set of defined gene variants within a tested patient population. The figure displays a "heat map" of gene variant frequencies that were identified within the population. Each vertical column marks 14 genes that were tested, some with multiple variants (three genes *CFP, C4BPA* and *CD59* were excluded from the "heat map" display due to the absence of these variants detected within this group of patients) Numerical list 1-42 (second row) correlates with the specific gene variants listed in Table 2. Each horizontal line represents one study subject, where each study subject was an hematopoietic stem cell transplant (HSCT) recipient. Heterozygous variants are shaded and homozygous variants are shown in black. This panel illustrates gene variants identified in all 77 HSCT recipients: 26 of 77 (34%) of subjects had at least one gene variant detected.
Figure 2 further illustrates the distribution of gene variants within the tested patient population in subjects with a diagnosis of TMA as compared to those without a diagnosis of TMA. The figure displays a "heat map" of gene variant frequencies that were identified within the population. Each vertical column marks 14 genes that were tested, some with multiple variants (three genes *CFP, C4BPA* and *CD59* were excluded from the "heat map" display due to the absence of these variants detected within this group of patients) Numerical list 1-42 (second row) correlates with the specific gene variants listed in Table 2. Each horizontal line represents one study subject, where each study subject was an HSCT recipient. Heterozygous variants are shaded and homozygous variants are shown in black. This figure shows that the majority of gene variants identified occurred in HSCT recipients with TMA (top panel, n=34), while few variants were seen in those without TMA (bottom panel, n=43). The median number of tested gene variants seen in recipients with TMA was 1 (0-7), and 0 (0-2) in those without TMA (p<0.0001).

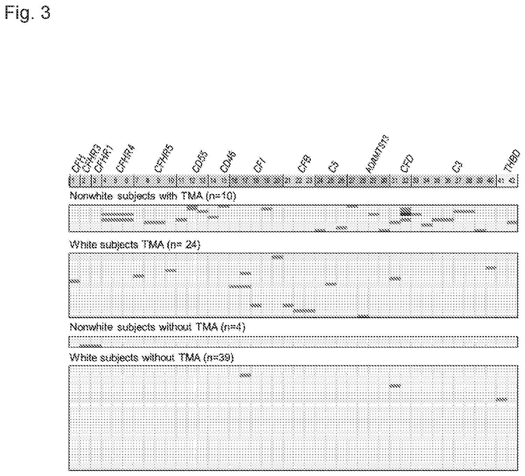

Figure 3 further illustrates the distribution of gene variants within the tested patient population. The figure displays a "heat map" of gene variant frequencies that were identified within the population. Each vertical column marks 14 genes that were tested, some with multiple variants (three genes *CFT, C4BPA* and *CD59* were excluded from the "heat map" display due to the absence of these variants detected within this group of patients) Numerical list 1-42 (second row) correlates with the specific gene variants listed in Table 2. Each horizontal line represents one study subject, where each study subject was an HSCT recipient. Heterozygous variants are shaded and homozygous variants are shown in black. This figure shows gene variant distribution separated by race and TMA. All ten (100%) non-whites with TMA had at least one variant detected, while 12/24 (50%) of white recipients had variants identified. Three or more (3-7) variants were only identified in non-white recipients with TMA (n=7); 5 of these 7 (71%) subjects died from severe TMA, indicating the effect of race and complement gene variants on TMA phenotype. *CFD* variant c.357+16C>A (marked as number 32, see Table 2) was identified exclusively in African-American HSCT recipients. Out of 12 whites with TMA who had gene variants identified, only three had two variants and other 9 had single variant detected. Only 3 of 39 non-whites without TMA (7.7%)) had only a single variant detected.

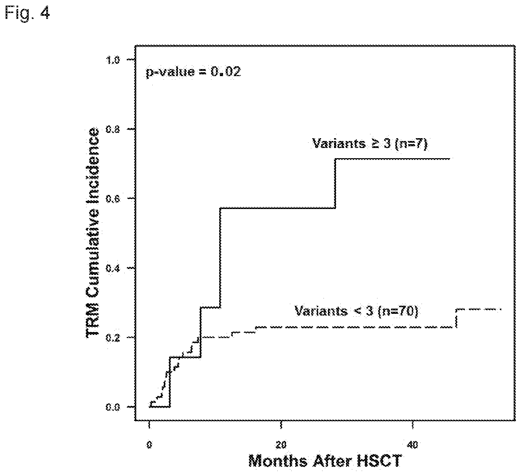

Figure 4 illustrates transplant related mortality in relation to the number of gene variants identified. Transplant related mortality (TRM, y-axis) is plotted as a function of time (x-axis). TRM in patients with 0-2 gene variants detected is given as a dashed line, while TRM in those with ≥3 gene variants detected is given as a solid line. TRM among study subjects was calculated using Kaplan-Meier method. The two groups were found to significantly differ using the log rank test, p=0.02. The TRM at 1 year and 3 years after HSCT was much higher in patients with ≥3 gene variants (57% at 1 year and 71% at 3 years) as compared to the patients with 0-2 variants (21% at 1 year and 24% at 3 years).

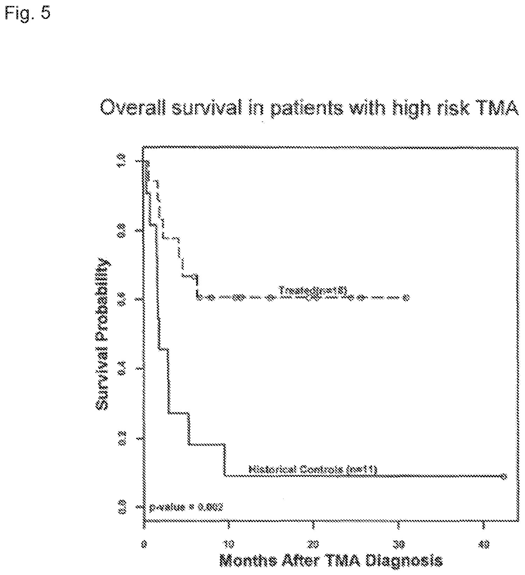

Figure 5 illustrates overall survival (Survival Probability, y-axis) over time (Months after TMA Diagnosis, x-axis) in eculizumab treated high-risk TMA patients (dashed line) vs. patients with high-risk features who did not receive eculizumab treatment (Historical Controls, solid line). High-risk features included nephrotic range proteinuria and elevated sC5B-9 levels. Kaplan-Meier and Log Rank tests were done starting at TMA diagnosis. Patients with high risk TMA who received eculizumab therapy had much better survival probability (p=0.002).

### DETAILED DESCRIPTION

Embodiments of the disclosure include systems, methods and compositions for prospective risk assessment and intervention to treat or prevent TMA, including in highly susceptible transplant recipients. The systems, methods and compositions described herein may be used to guide treatment strategies to improve outcomes, and may also be used to address, at least in part, racial disparities previously reported in transplant recipients. Nonetheless, the invention is defined in the appended claims.

Post-transplant thrombotic microangiopathy (TA-TMA) is a common complication of HSCT. However, due to its wide variation in severity, with many cases resolving spontaneously without intervention or with only supportive therapy, it is not routinely screened for until clinical symptoms emerge. At present, there are no methods to screen for TA-TMA prior to transplantation. Such a test would enable healthcare providers to direct observation to patients who are likely to develop TA-TMA in order to detect clinical symptoms at an earlier timepoint in the progression of the condition, leading to improved patient outcomes. Further, such a test would allow withdrawal of therapies that may trigger or exacerbate TA-TMA in favor of alternative treatments. Such a test may also allow the administration of prophylactic treatment for TA-TMA, or allow administration of treatments for TA-TMA at a timepoint in the progression of the condition that maximizes patient outcomes and reduces the morbidity and mortality associated with TA-TMA. Therefore, a test that can be administered to patients prior to HSCT is necessary in order to provide adequate observation and supportive care for HSCT patients. Such a test is also an integral part of methods to treat or prevent TATMA .

One embodiment is a system or method for determining the likelihood of an individual developing post-transplant thrombotic microangiopathy. These systems and methods may include (a) acquiring knowledge of the occurrence of one or more genetic variations associated with each member of a set of preselected biomarkers in the individual, wherein each member of the set of biomarkers is genetically associated with the complement pathway; (b) generating a personalized biomarker profile for the individual from the acquired knowledge; and (c) determining the risk that the individual will develop post-transplant thrombotic microangiopathy based at least in part upon the personalized biomarker profile. Embodiments may also include directing additional observation and/or treatment of the individual based on the individual's predicted risk of developing TA-TMA. In one embodiment, the biomarkers associated with the complement pathway include the genes shown in Table 1 below.

One embodiment is a system or method for determining the likelihood that an individual will develop post-transplant thrombotic microangiopathy subsequent to a stem cell transplant. In some embodiments, the system, methods and compositions described herein include a method for determining the risk that a subject will develop thrombotic microangiopathy subsequent to a hematopoietic stem cell transplant. Other embodiments include methods for predicting the severity of thrombotic microangiopathy in a subject subsequent to a hematopoietic stem cell transplant. Some further embodiments include a method for determining the prognosis for a subject that has developed, or is likely to develop, thrombotic microangiopathy subsequent to a hematopoietic stem cell transplant. Additional embodiments include methods for the treatment or prevention of thrombotic microangiopathy subsequent to a hematopoietic stem cell transplant.

Still other embodiments include systems, methods and compositions for directing the treatment of a subject that has developed, or is likely to develop, thrombotic microangiopathy subsequent to a hematopoietic stem cell transplant. In some embodiments, the methods and compositions described herein comprise a method for treating thrombotic microangiopathy subsequent to a hematopoietic stem cell transplant, including the step of prospectively assessing the risk and severity of the condition.

### Definitions

As used herein "subject" includes a vertebrate, including, but not limited to a mammal, a human, and a human patient. A "subject" may include a mammal, a human, or a human patient undergoing a transplant, including a hematopoietic stem cell transplant. As used herein, "subject" and "patient" may be used interchangeably.

As used herein a "sample" includes any sample obtained from a living system or subject, including fluid, blood, serum, tissue or cells. In some embodiments, a sample is obtained through sampling by minimally invasive or non-invasive approaches (e.g., urine collection, stool collection, blood drawing, needle aspiration, mucosal swab, saliva collection, and other procedures involving minimal risk, discomfort or effort). Samples can be solid (e.g., tissue), gaseous (e.g., exhaled breath), or liquid/fluid. Liquid samples include, but are not limited to, urine, blood, interstitial fluid, edema fluid, saliva, lacrimal fluid, inflammatory exudates, synovial fluid, abscess, empyema or other infected fluid, cerebrospinal fluid, sweat, pulmonary secretions (sputum), seminal fluid, feces, bile, intestinal secretions, and others. Samples also include a clinical sample such as serum, plasma, other biological fluid, or tissue samples. A sample may also include cells in culture, cell supernatants and cell lysates. Samples can be *in vivo* or *ex vivo.*

As used herein, the term "variant" or "gene variant" refers to a nucleic acid sequence or a peptide sequence that differs in sequence from a reference nucleic acid sequence or peptide sequence respectively, but retains essential properties of the reference molecule. Changes in the sequence of a nucleic acid variant may not alter the amino acid sequence of a peptide encoded by the reference nucleic acid, or may result in amino acid substitutions, additions, deletions, fusions and truncations. Changes in the sequence of peptide variants are typically limited or conservative, so that the sequences of the reference peptide and the variant are closely similar overall and, in many regions, identical. A variant and reference peptide can differ in amino acid sequence by one or more substitutions, additions, deletions in any combination. A variant of a nucleic acid or peptide can be a naturally occurring such as an allelic variant, or can be a variant that is not known to occur naturally. Non-naturally occurring variants of nucleic acids and peptides may be made by mutagenesis techniques or by direct synthesis.

As used herein, "prognosis" refers to the prospective determination of the likelihood of the occurrence of an event or condition, as well as an assessment of the likely severity of said event or condition. Such assessment of severity can include any clinical indicators, or predictors of morbidity, mortality, or other clinical outcome as would be known to one of ordinary skill in the art at the time the prognostic assessment was made. One of ordinary skill in the art will be presumed to be aware of the standard of care in the field at the time, as well as any foreseeable or obvious improvements to the standard of care, such as potential pharmaceutical, surgical, or other interventions which may ameliorate, exacerbate, or otherwise alter the course of the condition.

As used herein, the term "hematopoietic stem cell transplant" refers to the transplantation of stem cells derived from the bone marrow or peripheral blood or cord blood. Such transplant may be autologous, involving cells collected from the patient, or allogeneic, utilizing cells from a donor other than the patient. Such transplant may include the use of high dose chemotherapy or radiotherapy, including whole-body irradiation, to eliminate diseased cells prior to introduction of the transplant blood stem cells.

As used herein, Thrombotic Microangiopathy (TMA) refers to microangiopathic hemolytic anemia and platelet consumption, resulting in thrombosis and fibrin deposition in the microcirculation. As used herein, TMA can be diagnosed using published diagnostic criteria proposed by Cho et al.(2010) Transplantation 90(8):918-926 , and Jodele, S. et al. (2015) Blood Rev. 29(3):191-204. These criteria include but are not limited to (1) lactate dehydrogenase (LDH) elevated above the upper limit of normal for the subject's age; (2) de novo thrombocytopenia with a platelet count <50 × 10⁹/L or a ≥50% decrease in the platelet count; (3) de novo anemia with a hemoglobin below the lower limit of normal or anemia requiring transfusion support; (4) microangiopathic changes defined as the presence of schistocytes in the peripheral blood or histologic evidence of microangiopathy on a tissue specimen; (5) proteinuria, with a random urinalysis protein concentration of ≥ 30 mg/dL; (6) hypertension, comprising a blood pressure at the 95th percentile value for the patient's age, sex and height for pediatric patients, and a blood pressure ≥140/90 mm Hg in adults; (7) terminal complement activation as shown by an elevated plasma concentration of sC5b-9 above the upper normal laboratory limit; and (8) absence of a coagulopathy and a negative Coombs test, especially where ADAMTS13 activity is measured in subjects with TMA to exclude a diagnosis of thrombotic thrombocytopenic purpura. It is understood that one of ordinary skill in the art will be able to utilize alternative diagnostic criteria such as are known in, made obvious by, or made foreseeable by the state of the art at the time the clinical diagnosis is made.

As used herein, "clinical diagnosis" refers to the diagnosis of a physiological condition made with reference to observations of a subject's physical or physiological state, including reference to such laboratory tests as may be included within the state of the relevant art at the time the diagnosis is made.

As used herein, "treatment" (and grammatical variations thereof such as "treat" or "treating") refers to clinical intervention designed to alter the natural course of the individual or cell being treated during the course of clinical pathology. Desirable effects of treatment include, but are not limited to, decreasing the rate of disease progression, amelioration or palliation of the disease state, and remission or improved prognosis.

As used herein, "prevention" includes providing prophylaxis with respect to occurrence or recurrence of a disease or the symptoms associated with a disease in an individual. An individual may be predisposed to, susceptible to, or at risk of developing a disease, but has not yet been diagnosed with the disease.

As used herein, an "effective amount" or "therapeutically effective amount" refers to an amount of therapeutic compound, such as a complement inhibitor, administered to a mammalian subject, either as a single dose or as part of a series of doses, which is effective to produce a desired therapeutic effect.

As used herein, unless otherwise specified, "complement" or "the complement system" refers to the complement system of the mammalian innate immune system, for example, as described in Male, et al., (2006) Immunology (7th ed.) pp. 87-104, comprising at least the genes listed in Table 1 and the transcripts and products thereof.

As used herein, "complement modifying therapy" refers to any medication, procedure, lifestyle change, or other intervention that has the effect of changing the level of any component of the complement system such that the impact of the complement system on the cells, tissues, organs, or body of a patient is reduced, or of otherwise lessening the impact of the complement system on the cells, tissues, organs, or body of a patient. Such changes or reductions in impact may be effected either by direct interaction with a component of the complement system, or indirectly. A "complement activating therapy" will be understood to refer to any medication, procedure, lifestyle change, or other intervention that has the effect of maintaining or increasing the impact of the complement system on the cells, tissues, organs, or body of a patient, whether by directly or indirectly changing the level or activity of any component of the complement system or by other means known in the art.

As used herein, "screening" refers to any method of evaluating a gene including, without limitation, its sequence, expression level, methylation state, differential splicing, RNA editing, or chromatin state. Methods used for screening are those such as are disclosed below for determining and evaluating sequences for nucleic acids, as well as such methods as are known in the art for evaluation of gene expression, including, without limitation, RT-PCR and real-time RT-PCR; as well as methods for determining nucleic acid modification and chromatin state, such as chromatin immunoprecipitation, methylation analysis, and histone acetylation analysis.

One embodiment of the disclosure relates to the discovery that complement activation is correlated with development of TA-TMA in HSCT patients, and we have identified variants in 17 candidate genes known to play a role in complement activation. We have found that genetic mutations in these genes can be predictive for vascular injury and the severity of TMA after transplant. Here, we describe methods of pre-transplant genetic screening alone or in combination with functional tests that allow selection of high risk HSCT recipients who could benefit from specific interventions. This is particularly true where HSCT is a planned therapeutic procedure and transplant strategies can be modified depending on the genetic variations of these genes. For example, HSCT patients are often treated with calcineurin inhibitors and sirolimus to reduce rejection risk or prevent graft-vs.-host-disease (GVHD). However, these therapies are also associated with increased risk of TA-TMA. By determining TA-TMA risk prior to the HSCT procedure, alternative strategies for control of acute GVHD, such as *ex vivo* T-cell depletion or the use of photopheresis as prophylaxis can be applied in patients with elevated risk of TA-TMA.

By analyzing the genetic variants in these 17 complement activation genes and identifying high-risk patients for early therapeutic interventions before organ damage occurs, overall transplant outcomes should improve.

In some cases, complement inhibiting medicines, such as eculizumab (Alexion Pharmaceuticals, Cheshire, CT), other complement blocking agents, or complement modifying therapies such as plasmapheresis, can be initiated earlier with more reliable complement blockade that likely will result in further improvement of therapy response once a severe risk is discovered through discovering variants of the complement genes described below in Table 1.

Disclosed herein are systems and methods of determining the risk in a patient of developing TA-TMA after a hematopoietic stem cell transplant which include determining whether variants in any of the genes identified in Table 1 are present in the transplant recipient.

**Table 1:**

| **Gene Symbol** | **Description** | **Transcript/NCBI reference Number** | **AKA** |
|---|---|---|---|
| *CD46* | CD46 molecule, complement regulatory protein | NM_ 002389.4 | MCP |
| *THBD* | thrombomodulin | NM_ 000361.2 | |
| *CFH* | complement factor H | NM_000186.3 | |
| *CFI* | complement factor I | NM_ 000204.3 | |
| *CFB* | complement factor B | NM_ 001710.5 | |
| *C3* | complement component 3 | NM_ 000064.2 | |
| *C5* | complement component 5 | NM_001735.2 | |
| *CFD* | complement factor D | NM_ 001928.2 | |
| *CFHR1* | complement factor H-related 1 | NM_ 002113.2 | |
| *CFHR3* | complement factor H-related 3 | NM_ 021023.5 | CFHL |
| *CFHR4* | complement factor H-related 4 | NM_ 006684.4 | |
| *CFHR5* | complement factor H-related 5 | NM_ 030787.3 | |
| *CFP* | complement factor properdin | NM_ 002621.2 | |
| *C4BPA* | complement component 4 binding protein, alpha | NM_ 000715.3 | |
| *CD59* | CD59 molecule, complement regulatory protein | NM_ 203330.2 | |
| *CD55* | CD55 molecule, decay accelerating factor for complement | NM_ 000574.3 | |
| *ADAMTS13* | ADAM metallopeptidase with thrombospondin type 1 motif, 1 3 | NM_ 139025.3 | |

Such variants include, but are not limited to, those listed in Table 2:

**Table 2:**

| No* | RS# | Gene | Chr^{&} | cDNA | Amino Acid | MAF (EA) | MAF (AA) |
|---|---|---|---|---|---|---|---|
| 1 | N/A | *CFH* | 1 | c.1281C>A | p.T427T | N/A | N/A |
| 2 | rs144398879 | *CFHR3* | 1 | c.354C>A | p.Y118* | 0 | 0.0045 |
| 3 | rs146147352 | *CFHPI* | 1 | c.130C>T | p.Q44* | 0 | 0.003 |
| 4 | rs78624607 | *CFHR4* | 1 | c.62T>C | p.V21A | 0 | 0.0242 |
| 5 | rs78951215 | *CFHR4* | 1 | c.159C>T | p.S53S | 0 | 0.0217 |
| 6 | rs77556138 | *CFHR4* | 1 | c.243G>A | p.T81T | 0 | 0.0289 |
| 7 | rs185709089 | *CFHR5* | 1 | c.254-5C>T | N/A | 0.0038 | 0.0009 |
| 8 | N/A | *CFHR5* | 1 | c.486_487insA | p.E163Rfs*35 | 0.0073 | 0.0014 |
| 9 | rs41299613 | *CFHR5* | 1 | c.622T>C | p.C208R | 0.0022 | 0.0009 |
| 10 | N/A | *CFHR5* | 1 | c.1446T>C | p.Y482Y | N/A | N/A |
| 11 | rs7542430 | *CD55* | 1 | c.6C>G | p.T2T | 0 | 0.0077 |
| 12 | rs28371603 | *CD55* | 1 | c.294C>T | p.C98C | 0.0005 | 0.0102 |
| 13 | rs60822373 | *CD55* | 1 | c.679G>C | p.A227P | 0.0003 | 0.0195 |
| 14 | rs17006830 | *CD46* | 1 | c.796G>A | p.D266N | 0 | 0.0123 |
| 15 | rs41317833 | *CD46* | 1 | c.971C>T | p.P324L | 0 | 0.0068 |
| 16 | rs113460688 | *CFI* | 4 | c.1657G>A | p.P553S | 0.0017 | 0.0007 |
| 17 | rs114013791 | *CFI* | 4 | c.1534+5C>A | N/A | 0.0151 | 0.0039 |
| 18 | rs41278047 | *CFI* | 4 | c.1322T>C | p.K441R | 0.0052 | 0.0005 |
| 19 | rs61733901 | *CFI* | 4 | c.1246T>G | p.I416L | 0.0001 | 0.0129 |
| 20 | rs74817407 | *CFI* | 4 | c.1217C>T | p.R406H | 0.0009 | 0.0018 |
| 21 | rs45484591 | *CFB* | 6 | c.1697A>C | p.E566A | 0.0078 | 0.0066 |
| 22 | rs199518433 | *CFB* | 6 | c.1729G>A | p.V577I | 0.0002 | 0 |
| 23 | rs200095748 | *CFB* | 6 | c.2005G>C | p.V669L | 0.0002 | 0 |
| 24 | N/A | *C5* | 9 | c.3732C>T | p.T1244T | N/A | N/A |
| 25 | N/A | *C5* | 9 | c. 3231-4T>C | N/A | N/A | N/A |
| 26 | rs62640871 | *C5* | 9 | c.921G>C | p.V307V | 0 | 0.005 |
| 27 | rs115943536 | *ADAMTS13* | 9 | c.1022C>T | p.P341L | 0.0001 | 0.0233 |
| 28 | N/A | *ADAMTS13* | 9 | c.1225C>T | p.R409W | N/A | N/A |
| 29 | rs36220239 | *ADAMTS13* | 9 | c.1368G>T | p.Q456H | 0.0001 | 0.0495 |
| 30 | rs78977446 | *ADAMTS13* | 9 | c.2708C>T | p.S903L | 0 | 0.0002 |
| 31 | rs35186399 | *CFD* | 19 | c.205G>A | p.E69K | 0.0076 | 0.0013 |
| 32 | rs116135466 | *CFD* | 19 | c.357+16C>A | N/A | 0.0005 | 0.0922 |
| 33 | rs144589541 | *C3* | 19 | c.4767C>T | p.K1589K | 0.0001 | 0.0027 |
| 34 | N/A | *C3* | 19 | c.4635G>A | p.Y1545Y | N/A | N/A |
| 35 | rs140143815 | *C3* | 19 | c.1722C>T | p.Q574Q | 0.0002 | 0 |
| 36 | N/A | *C3* | 19 | c.1243G>T | p.P415T | N/A | N/A |
| 37 | rs138833296 | *C3* | 19 | c.1044G>A | p.I348I | 0 | 0.0002 |
| 38 | rs202057992 | *C3* | 19 | c.876+11G>T | N/A | 0 | 0.0011 |
| 39 | rs3745558 | *C3* | 19 | c.600-14G>A | N/A | 0.0005 | 0.0043 |
| 40 | rs147859257 | *C3* | 19 | c.463T>G | p.K155Q | 0.0043 | 0.0002 |
| 41 | rs1800578 | *THBD* | 20 | c.1483G>A | p.P495S | 0.0014 | 0 |
| 42 | rs41400249 | *THBD* | 20 | c.1208C>T | p.R403K | 0 | 0.0007 |

| | | | | | | | |
|---|---|---|---|---|---|---|---|
| * RS#: reference SNP cluster ID; & Chromosome; MAF (EA): Minorallele frequency in European-American based on data from the EVS database (NHLBI Exome Sequencing Project); MAF (AA): Minor allele frequency in African-American based on data from the EVS database (NHLBI Exome Sequencing Project). | | | | | | | |

One embodiment is a method for determining the likelihood of an individual developing post-transplant thrombotic microangiopathy by (a) acquiring knowledge of the occurrence of one or more genetic variations associated with each member of a set of preselected biomarkers in the individual, wherein each member of the set of biomarkers is genetically associated with the complement pathway; (b) generating a personalized biomarker profile for the individual from the acquired knowledge; and (c) determining the likelihood that the individual will develop post-transplant thrombotic microangiopathy based at least in part upon the personalized biomarker profile.

In some embodiments, the set of preselected biomarkers comprises variants in the set of genes disclosed in Table 1. In some further embodiments, the set of preselected biomarkers comprises the gene variants disclosed in Table 2.

In some embodiments, the method comprises determining the gene variants in an individual for all 17 of the genes disclosed in Table 1. In further embodiments, the method comprises screening any 16 of the genes disclosed in Table 1 for possible variations. In further embodiments, the method comprises screening any 15, 14, 13, 12, 11, 10, 9, 8, 7, 6, 5, 4, 3, 2, or one of the genes disclosed in Table 1. It is further understood that one of ordinary skill in the art would recognize additional loci, including additional complement-related genes, as being loci in which genetic variants would foreseeably have equivalent predictive value with respect to TA-TMA risk.

Some embodiments include determining that the risk of developing TA-TMA in a patient by finding no or few relevant variants in the complement-associated genes. This would signal that the individual has a lower risk of developing TA-TMA or a lower risk that the individual have a severe form of TA-TMA. A finding of one relevant variant in the list of complement-associated genes may signal an elevated risk of developing TA-TMA or an elevated risk that the individual have a severe form of TA-TMA. A finding of two to three variants in the complement-associated genes may signal a higher risk of developing TA-TMA or a higher risk that the individual have a severe form of TA-TMA. A finding that three or more relevant variants in the complement-associated genes may signal a high risk that the individual will develop TA-TMA or a high risk that the individual have a severe form of TA-TMA. In some embodiments, the relevant variants are those variants disclosed in Table 2.

In some embodiments, the method of determining the genetic variations associated with the complement-associated genes is performed by obtaining a biological sample from the subject, and subjecting the genetic material within the sample to analysis by nucleotide sequencing. In one embodiment, the nucleotide sequencing may be performed by well-known next-generation sequencing machines manufactured by Illumina (San Diego, CA) or Thermo Fisher (San Diego, CA). Other ways of determining the variants in complement-related genes are also contemplated. For example, a biological sample can be analyzed by polymerase chain reaction (PCR), reverse-transcription PCR (RT-PCR), real-time RT-PCR; single-strand conformational polymorphism (SSCP) analysis or microarray analysis. Additional techniques may include subtractive hybridization or hybridization analysis, including by fluorescent hybridization and/or fluorescence resonance energy transfer (FRET) analysis. Any other method known to those within the art for the identification of polymorphisms in genetic loci are contemplated by the present application.

In some embodiments, probes are provided, comprising molecules capable of selectively identifying relevant loci or distinguishing between variants. Such probes may include, without limitation, ribonucleic acid molecules, deoxyribonucleic acid molecules, peptide nucleic acid molecules, branched nucleic acid molecules, nucleic acid mimetics, and any other molecules capable of identifying specified loci or distinguishing sequence variants. In some embodiments, such probes may include artificial sequence tags. In some embodiments, the probes may comprise primers for PCR or sequencing. In some embodiments, the probes may be bound to a substrate. In some embodiments, the probes may incorporate a radiolabel, colorimetric label, fluorescent label, spin label, spectroscopic label, or other method of permitting detection of the probe.

As mentioned above, in some embodiments, a system of the disclosure comprises an array system. Non-limiting examples of arrays include microarrays, bead arrays, through-hole arrays, well arrays, and other arrays known in the art suitable for use in hybridizing probes to targets. Arrays can be arranged in any appropriate configuration, such as, for example, a grid of rows and columns. Some areas of an array comprise the sequence variant detection probes whereas other areas can be used for image orientation, normalization controls, signal scaling, noise reduction processing, or other analyses. Control probes can be placed in any location in the array, including along the perimeter of the array, diagonally across the array, in alternating sections or randomly. In some embodiments, the control probes on the array comprise probe-pairs of sense and antisense probes. The number of control probes can vary, but typically the number of control probes on the array range from 1 to about 500,000. In some embodiments, at least 10, 100, 500, 1,000, 5,000, 10,000, 25,000, 50,000, 100,000, 250,000 or 500,000 control probes are present. When control probe pairs are used, the probe pairs will range from 1 to about 250,000 pairs. In some embodiments, at least 5, 50, 250, 500, 2,500, 5,000, 12,500, 25,000, 50,000, 125,000 or 250,000 control probe pairs are present. The arrays can have other components besides the probes, such as linkers attaching the probes to a support. In some embodiments, materials for fabricating the array can be obtained from Affymetrix (Santa Clara, California), GE Healthcare (Little Chalfont, Buckinghamshire, United Kingdom) or Agilent Technologies (Palo Alto, California).

Besides arrays where probes are attached to the array substrate, numerous other technologies may be employed in the disclosed system for the practice of the methods of the disclosure. In one embodiment, the probes are attached to beads that are then placed on an array as disclosed by Ng et al. (Ng et al. (2008) Biosensors & Bioelectronics 23:803-810).

In another embodiment, probes are attached to beads or microspheres, the hybridization reactions are performed in solution, and then the beads are analyzed by flow cytometry, as exemplified by the Luminex multiplexed assay system. In this analysis system, homogeneous bead subsets, each with beads that are tagged or labeled with a plurality of identical probes, are combined to produce a pooled bead set that is hybridized with a sample and then analyzed in real time with flow cytometry, as disclosed in US Patent 6,524,793. Bead subsets can be distinguished from each other by variations in the tags or labels, e.g., using variable in laser excitable dye content.

In a further embodiment, probes are attached to cylindrical glass microbeads as exemplified by the Illumina® Veracode multiplexed assay system. Here, subsets of microbeads embedded with identical digital holographic elements are used to create unique subsets of probe-labeled microbeads. After hybridization, the microbeads are excited by laser light and the microbead code and probe label are read in real time multiplex assay.

In another embodiment, a solution based assay system is employed as exemplified by the NanoString nCounter Analysis System (Geiss, G. et al. (2008) Nature Biotechnol. 26:317-325). With this methodology, a sample is mixed with a solution of reporter probes that recognize unique sequences and capture probes that allow the complexes formed between the nucleic acids in the sample and the reporter probes to be immobilized on a solid surface for data collection. Each reporter probe is color-coded and is detected through fluorescence.

In a further embodiment, branched DNA technology, as exemplified by Panomics QuantiGene Plex 2.0 assay system, is used. Branched DNA technology comprises a sandwich nucleic acid hybridization assay for RNA detection and quantification that amplifies the reporter signal rather than the sequence. By measuring the RNA at the sample source, the assay avoids variations or errors inherent to extraction and amplification of target polynucleotides. The QuantiGene Plex technology can be combined with multiplex bead based assay system such as the Luminex system described above to enable simultaneous quantification of multiple RNA targets directly from whole cells or purified RNA preparations.

In some embodiments, the methods and compositions described herein comprise a method for preventing TA-TMA in an HSCT recipient, including the steps of 1) determining an individual's risk of developing TA-TMA prior to, during, or subsequent to transplant, and 2) in individuals with elevated or high TA-TMA risk, withdrawing therapies known to trigger or exacerbate complement activation. Such therapies include, but are not limited to, calcineurin inhibitors and sirolimus (rapamycin). In addition, alternatives to such therapies may be included to reduce the potential for graft-versus-host disease (GVHD), including without limitation, *ex-vivo* T-cell depletion and photopheresis. In other embodiments, alternative therapies for preventing GVHD can be implemented and/or therapies known to trigger or exacerbate complement activation can be withheld until after an individual's risk of developing TA-TMA has been ascertained. In still further embodiments, the determination of an individual's risk of developing TA-TMA can be ascertained during a transplant procedure. In still further embodiments, the determination of an individual's risk of developing TA-TMA can be ascertained subsequent to a transplant procedure.

In some embodiments, the methods and compositions described herein comprise a method for treating TA-TMA in an HSCT recipient, including the steps of determining an individual's risk of developing TA-TMA prior to transplant, and administering one or more complement modifying therapies to patients at elevated risk of developing TA-TMA. Such complement modifying therapies may include, without limitation, Rituximab, Eculizumab, and purified human C1 esterase inhibitor. Such complement modifying therapies may further include, without limitation, procedures such as plasmapheresis.

In some embodiments, the methods and compositions described herein comprise a method for directing the treatment of an HSTC recipient, including the steps of determining the likelihood of an individual to develop post-transplant thrombotic microangiopathy and using the risk profile developed to direct health care providers to observe subjects at elevated risk of developing TA-TMA for early clinical symptoms of TA-TMA. In some further embodiments, the method directs that individuals determined by the methods herein to be at low risk for TA-TMA (for example, those possessing none of the identified variants given in Table 2 or their equivalents) be subjected to limited general observation for the development of TA-TMA symptoms; while individuals determined by the methods herein to be at elevated risk for TA-TMA (for example, those possessing one of the identified variants given in Table 2 or their equivalents) are directed to be observed closely, including the administration of all necessary clinical and laboratory tests, immediately following the HSCT procedure for the development of TMA symptoms and treated immediately upon onset of those symptoms; patients determined by the methods herein to be at high risk of developing TA-TMA (for example, those possessing one or two of the identified variants given in Table 2 or their equivalents) are directed be prevented from embarking on a course of sirolimus or calcineurin inhibitor therapy in favor of alternative methods of reducing GVHD risk; and patients determined by the methods disclosed herein to be at very high risk (for example, those possessing two, three, or more of the identified variants given in Table 2 or their equivalents) are directed to be treated with a complement blocking medication such as eculizumab in addition to utilizing alternatives to sirolimus or calcineurin inhibitor therapy.

In some embodiments the compositions and methods described herein comprise a kit for determining the risk of developing TA-TMA in a subject as well as determining the prognosis for the likely severity of TA-TMA in a subject. In some embodiments, such a kit comprises oligonucleotide primers for the PCR amplification of genomic regions associated with TA-TMA. In some embodiments, such a kit comprises oligonucleotide primers for the PCR amplification of the sequences corresponding to the genes named in Table 1. In some embodiments such a kit comprises labeled nucleotide probes for the detection of sequence variants associated with TA-TMA. In some embodiments such a kit comprises nucleotide probes for the detection of sequence variants associated with TA-TMA bound to a substrate. In some embodiments, the kit comprises an array of nucleotide probes for the detection of sequence variants associated with TA-TMA. In some embodiments, such a kit comprises PCR primers, labeled probes, substrate-bound probes, and/or other compositions for the detection of all 17 of the sequences identified in Table 1. In some embodiments, such a kit comprises PCR primers, labeled probes, substrate-bound probes, or other compositions for the detection of any 16, any 15, any 14, any 13, any 12, any 11, any 10, any 9, any 8, any 7, any 6, any 5, any 4, or any 3 of the sequences identified in Table 1. In some embodiments, such a kit comprises instructional materials directing the treatment of a subject in accord with the methods described herein in response to the risk determination provided through use of the kit.

### EXAMPLE 1

To further explore the mechanism of TMA, we performed a prospective analysis of 17 candidate genes known to play a role in complement activation. *See* Jodele S, et al. (2015) Blood Nov 24. pii: blood-2015-08-663435. (Epub ahead of print).

### Study Subjects

One hundred consecutive patients who underwent HSCT at Cincinnati Children's Hospital Medical Center (CCHMC) from September 2010 until December of 2011 were enrolled onto a prospective TMA biomarker study, after approval from the institutional review board. Thirty nine percent of subjects met criteria for TMA using prospective monitoring as previously described (Jodele S, et al. (2014) Blood. 124(4):645-653). TMA diagnostic criteria included: (1) lactate dehydrogenase (LDH) above the upper limit of normal; (2) de novo thrombocytopenia with a platelet count <50x109/L or a ≥50% decrease in the platelet count; (3) de novo anemia with a hemoglobin below the lower limit of normal or anemia requiring transfusion support; (4) microangiopathic changes defined as the presence of schistocytes in the peripheral blood or histologic evidence of microangiopathy; and (5) absence of a coagulopathy and a negative Coombs test. ADAMTS 13 activity was measured at TMA presentation time to exclude diagnosis of thrombotic thrombocytopenic purpura (TTP). The date of TMA diagnosis was defined as the first date when all diagnostic criteria were fulfilled. There were 90 allogeneic transplant recipients in this study and those with adequate genomic pre-transplant DNA (n=77) participated in genetic analyses. Outcomes analyses to examine the impact of race on survival and occurrence of TMA were performed in an expanded cohort of the most recent 333 consecutive HSCT recipients transplanted at CCHMC. The study was conducted in accordance with the Declaration of Helsinki.

### Genetic Testing

Genomic DNA was isolated from recipients' blood prior to transplantation. Seventeen genes involved in pathogenesis of other thrombotic microangiopathies were selected for testing. See Meri S (2013) Eur J Intern Med. 24(6):496-502, Bu F, et al. (2014) J Am. Soc. Nephrol. 25(1):55-64, Feng S, et al. (2013) Blood 122(8): 1487-1493, Bresin E, et al. (2013) J Am Soc Nephrol. 24(3):475-486, Crovetto F, et al. (2012) J Matern. Fetal Neonatal Med. 25(11):2322-2325, and Jodele S, et al. (2013) Blood. 122(12):2003-2007. Genes tested were: *CFH, CPHR1, CFHR3, CFHR4, CPHR5, CD55, CD59, CD46, CFI, CFB, CFP, C5, ADAMTS13, CFD, C3, C4BPA and THBD.* All exons, flanking intronic and untranslated regions (5' and 3') were enriched/captured using microdroplet PCR technology (RainDance Technologies Inc., USA). Enriched targets were sequenced using next generation sequencing technology on the Illumina HiSeq 2500 instrument (Illumina Inc., USA) with > 20-fold coverage at every target base. Resulting sequence reads were aligned against reference DNA sequence and variants were analyzed using NextGENe software (SoftGenetics, LLC, USA). Observed variants were compared against dbSNP (NCBI). Novel variants were further evaluated using laboratory developed bioinformatic tools (Sivakumaran TA, et al. (2013) Otolaryngol. Head Neck Surg. 148(6): 1007-1). Variants were filtered by focusing on coding, frame shift (indels), nonsense and splicing modifying changes. Rare variants were defined as variants with a minor allele frequency less than 1% in European-American or African-American populations based on data from the EVS database (NHLBI Exome Sequencing Project). Predicted pathogenic mutations are variants with high pathogenicity predictions using in silico analyses including SIFT, PolyPhen2, MutationTaster, ALIGN GVGD and Human Splicing Finder splice site prediction algorithm. All identified variants were confirmed by Sanger sequencing (Applied Biosystem). In addition, deletions at the *CFHR3*/*CFHR1* loci were detected using multiple ligation-dependent probe amplification (MLPA) method (MRC Holland, Amersterdam, Netherlands). For completeness, we also included synonymous variants. These otherwise neutral for amino acid sequence variants could create a cryptic spicing site and represent unique haplotypes that are important to the susceptibility to TMA, or they could regulate transcription or translation.

### RNAseq Analysis

RNAseq was performed by Genomics, Epigenomics and Sequencing Core (GESC) in the University of Cincinnati on pre-transplant whole blood mononuclear cells (MNCs) from eight HSCT recipients with TMA who had identified gene variants and eight HSCT recipients without TMA and without any identified gene variants based on sample availability. We selected pre-transplant RNA (presumed resting state) for this assay because we wished to examine the impact of genotype, not the possible effects of activation of complement during the transplant process. Expression of each gene with a genetic variant, and of pathways related to complement activation, was compared with expression of the same gene in HSCT subjects with and without TMA.

The RNA-sequencing of 50 nucleotide long single end reads was performed using standard Illumina protocol on the Illumina HiSeq system. Sequence reads were aligned to the reference human genome using the TopHat aligner (Trapnell C, et al. (2009) Bioinformatics. 25(9):1105-1111), and reads aligning to each known transcript were counted using Bioconductor packages for next-generation sequencing data analysis. See Huber W, et al. (2015) Nat Methods. 12(2): 115-121. The differential expression analysis between TMA samples with variants in a specific gene and non-TMA samples without variants was performed for each gene separately based on the negative-binomial statistical model of read counts as implemented in the DESeq Bioconductor package (Anders S, et al. (2013) Nat Protoc. 8(9):1765-1786). The pathway enrichment analysis was performed separately for upregulated and downregulated genes using the LRpath methodology (Sartor MA, et al. (2009) Bioinformatics. 25(2):211-217) and the MSigDB database of gene lists and pathways (Subramanian A, et al. (2005) Proc Natl Acad Sci U S A. 102(43):15545-15550). The enrichment p-values were adjusted for testing all pathways in MSigDB using the false discovery rates (Storey JD and Tibshirani R. (2003) Proc Natl Acad Sci U S A. 100(16):9440-9445).

### Statistical Analysis

Median (range) and frequencies were reported to describe continuous and categorical variables respectively. Differences by group for continuous and categorical variables were compared using Fisher exact and Wilcoxon tests, respectively. Survival and competing risk analyses were performed using the Kaplan-Meier method. Log-rank tests were used to assess the difference in overall survival by group. Gray's method was used to test for difference in cumulative incidence by group. For cumulative incidences death by relapse or all-cause mortality were considered competing risks in the analysis of non-relapse mortality (NRM) and TMA, respectively. Analyses were performed using R version 3.1.3. All statistical tests were two-sided and significance was assessed at p<0.05.

### Results

### Study demographics and patient characteristics

Seventy seven of the 90 allogeneic transplant recipient enrolled on the study had adequate pre-transplant DNA for analysis. Thirty four of these HSCT recipients had a clinical diagnosis of TMA based on prospective screening, and 43 did not. Study subjects were children younger than 18 years of age, and the majority were white males. A majority of patients (66%) had HSCT for non-malignant disorders including bone marrow failure or immunodeficiency. Stem cell source was mainly bone marrow (78%) from unrelated donors (73%). A similar number of patients received myeloablative and reduced intensity regimens (53% vs 47%).

Even though non-white transplant recipients composed only 18% of our total study cohort, a significantly higher fraction of non-whites than whites had TMA (71% vs 39%, p=0.04). All transplant recipients received cyclosporine (CSA) for graft versus host disease (GVHD) prophylaxis. Uniform drug monitoring was used to maintain a CSA trough of 250 to 350 ng/mL. There was no difference in CSA levels between patients who developed TMA and those who did not (251.6 and 247.2 ng/mL, p=0.65). Children with Fanconi anemia (FA) had increased incidence of TMA. These were the only cases who received both T-cell-depleted PBSCs and CSA, so we were not able to tell whether the increased risk of TMA reflected increased susceptibility in persons with FA or was associated with the treatment protocol itself.

Similarly, children with malignant diagnoses received CSA and methotrexate for GVHD prophylaxis. Both malignancy and receiving methotrexate were associated with lower incidence of TMA, so we could not identify whether diagnosis or treatment regimen influenced TMA incidence. None of the study subjects received any targeted therapy (e.g. complement blockade) for TMA.

### Gene variants identified

A total of 239 variants in 17 genes were identified after initial quality control (>20-fold coverage and false positive elimination by visual inspection and Sanger sequencing). Three genes *CFP, C4BPA* and *CD59* were not further considered because no variants were identified. After excluding deep intronic and untranslated regions (UTR) variants (n=58) and common single nucleotide polymorphisms (SNPs) (n=139), we identified 42 likely functional variants, including 15 previously reported microangiopathy-related variants. Of the 15 previously reported variants, six are known to be pathogenic and nine are variants with uncertain clinical significance (VUCS). Of the 27 previously unreported variants two are predicted pathogenic, 10 are considered VUCS and 15 are predicted likely benign by computer methods for predicting functional consequences of sequence variants. We performed pathogenicity predictions using SIFT, PolyPhen2, MutationTaster, ALIGN GVGD and Human Splicing Finder splice site prediction algorithms to evaluate the 27 variants not previously described (two nonsense, nine missense, 12 synonymous and three splice site variants). Two nonsense variants in *CFHR1* and *CFHR3* were considered pathogenic by nature of predicted effects, and 15 variants, including synonymous and splice site variants, were predicted to be likely benign. Since predictive algorithms are of limited accuracy, considered by many to be correct only about 70% of the time, we did not want to overlook important changes for predicted benign variants so these were retained in the analysis and gene expression profiling was performed on available samples (see RNAseq analysis).

Thirty four percent of tested subjects (26/77) had at least one gene variant identified (Figure 1). Figure 2 displays a "heat map" of gene variant distribution stratified according to presence or absence of TMA after HSCT, showing significantly higher frequency of variants in patients with TMA. Twenty-two of 34 (65%) subjects with TMA had at least one gene variant identified, as compared to four of 43 (9%) subjects without TMA (p<0.0001). Pathogenic variants previously described in other microangiopathies were seen in *CFHR5, CFI* and *ADAMTS13,* all in recipients with TMA. ADAMTS13 activity tested at TMA diagnosis was slightly reduced (43-50%) in subjects with *ADAMTS13* variants, but did not meet criteria for diagnosis of TTP. Three *CFI* variants seen in our study have been previously reported in aHUS, two *ADAMTS13* variants in TTP, and one *CFHR5* variant is known to cause *CFH-*related protein deficiency. See Vernon, K.A., et al. (2012) Am. J. Kidney Dis. 60(1): 121-125; Caprioli, J, et al. (2006) Blood 108(4): 1267-1279; Camiller, R.S., et al. (2012) J. Thromb. Haemost. 10(9):1792-1801; Liu F., et al. (2005) Zhonghua Xue Ye Xue Za Zhi. 26(9):521-524; and Cayci, F.S., et al. (2012) Pediatr. Nephrol. 27(12):2327-2331.

A *CFD* variant c.357+16C>A was only detected in African-American patients with TMA. The median number of gene variants seen in recipients with TMA was one (range 0-7), and zero (range 0-2) in those without TMA (p<0.0001). In addition to NGS results, two recipients with TMA had homozygous deletion of *CFHR3*/*R1* detected by MLPA that was not seen in recipients without TMA. See Skerka , C., et al. (2010) Semin. Thromb. Hemost. 36(6):625-632. Both patients with homozygous deletion of *CFHR3*/*R1* had severe multi-visceral TMA and died. No known pathogenic variants were seen in recipients without TMA. The high frequency of variants in recipients with TMA compared to those without TMA (distributed by the "selective pressure" of transplant) suggests that the variants are associated with TMA and do not represent random polymorphic variation in the genes tested.

In our study cohort evaluated for genetic variants (n=77) the cumulative incidence of TMA was higher in non-whites than whites (71% vs 39 %, p = 0.04). Moreover, more gene variants were detected in non-white than white transplant recipients: 2.5 (0-7) vs. 0 (0-2), p<0.0001. Figure 3 shows a "heat map" of gene variants stratified by race and TMA, illustrating that the highest number of variants occurred in non-white patients with TMA.

Figure 4 shows the association of gene variant number with transplant related mortality. Seven patients with TMA, all non-whites, had each between three and seven variants identified (three variants each (n=3), four variants each (n=2), seven variants each (n=2). Five of these seven non-white patients with ≥3 gene variants died from severe TMA, indicating an important association of race and cumulative effect of complement gene variants with TMA phenotype. Only one of the four non-white recipients without TMA had two variants detected. None of the white recipients without TMA had more than two variants detected.

To address whether gene variants were associated with race versus TMA, we analyzed only white HSCT recipients. Fifty percent (12/24) of whites with TMA had at least one variant, compared with 7.7% (3/39) of whites without TMA. Eighty percent of white patients with at least one gene variant developed TMA (12 of 15) compared with 25% (12 of 48) of white patients without any variant (p<0.0001). This observation indicates that the presence of gene variants is significantly enriched in subjects of any race who develop TMA, but non-whites have more variants and more severe disease.

Next, we compared outcomes in recipients who had 0-2 or ≥3 gene variants. Patients with ≥3 gene variants, who were all non-whites, had increased transplant related mortality (TRM) (57%; vs 21% at 1y; p=0.02) (Figure 3). These data led us to hypothesize that TRM would be elevated, and survival reduced in non-whites transplanted at our center. We analyzed an expanded cohort of 333 consecutive transplants performed at CCHMC, comparing survival in patients with and without TMA, in white and non-white recipients. This larger cohort included 48 non-white recipients, most of whom (85%) were African Americans. The cumulative incidence of TMA in this cohort was 35% (40% in allogeneic transplant recipients and 8% in autologous, p<0.0001) and again was significantly more frequent in non-white than white HSCT recipients (52% vs 29%p= 0.008). Supporting our hypothesis, non-white recipients had worse survival than whites (56% vs 81% at 1y, p = 0.0009) and HSCT recipients with TMA had inferior survival compared to those without TMA (63% vs 85% at ly, p=0.0001). Furthermore, non-white patients with TMA had significantly worse survival after transplantation than whites with TMA (37% vs 69% at ly, p=0.04), while survival was not different by race in subjects without TMA (73% versus 87% at 1y, p=0.12), supporting our hypothesis that at least a proportion of the inferior survival seen in non-white HSCT recipients is mediated by increased susceptibility to severe TMA.

We evaluated the functional importance of genetic variants using RNAseq, comparing gene expression in peripheral blood collected prior to transplantation from eight transplant recipients with high numbers of gene variants and TMA and eight recipients who did not have any variants identified and did not develop TMA. The pathway analysis of differentially expressed genes indicated up-regulation of complement-associated pathways in subjects with variants compared with those without. Importantly, we observed up-regulation of complement pathways even in persons with variants predicted benign by some algorithms. Of particular interest, a *C5* variant c.921G>C (Table 2, #26) that is predicted to be benign was the only detected variant in the subject with TMA and showed multiple complement pathway upregulation supporting a possible role in disease. The analysis provided no evidence of down-regulation of complement pathways in any subjects with gene variants. These data support our hypothesis that identified gene variants may facilitate rapid complement activation.

### Summary

Among 77 subjects undergoing genetic testing, 34 had TMA. Sixty five percent of patients with TMA had genetic variants in at least one of the 17 complement related genes in Table 1, as compared to 9% of patients without TMA (p<0.0001), Gene variants were increased in subjects with TMA of all races, but non-whites had more variants than whites (2.5 (0-7) vs 0 (0-2), p<0.0001). Variants in ≥3 genes were identified only in non-whites with TMA, and were associated with high mortality (71%). RNA sequence analysis of pre-transplant samples showed upregulation of multiple complement pathways in subjects with TMA who had one or more of the tested gene variants, as compared to those without TMA and lacking the tested gene variants. Our data revealed important differences in genetic susceptibility to HSCT-associated TMA based on recipient genotype.

It is possible that some of the identified genetic variants may not have biological importance in the course of normal life, but under the stress of HSCT, with significant vascular injury caused by intense radiation and/or chemotherapy, these susceptible individuals may manifest TMA.

Calcineurin inhibitors and sirolimus are associated with increased risk of TMA. Alternative strategies for control of acute GVHD, for example, *ex vivo* T-cell depletion or use of photopheresis as prophylaxis could be used and novel complement blockers might be a therapeutic option in high-risk patients. Close monitoring of high-risk patients and early therapeutic interventions, before organ damage occurs, should improve overall transplant outcomes. Combining appropriate use of eculizumab or other complement inhibiting medications in this clinical setting, with the use of TMA biomarkers that predict poor outcome, therapy can be initiated earlier with more reliable complement blockade that likely will result in further improvement of therapy response.

### EXAMPLE 2

Eighteen patients showing high risk features for TA-TMA, including nephrotic range proteinuria and elevated sC5b-9 levels, were treated with eculizumab (Alexion Pharmaceuticals, Cheshire, CT). Dosing levels were adjusted to maintain a therapeutic eculizumab concentration of 100ug/ml. *See* Jodele S. et al. (2015), Biol. Blood Marrow Transplant. pii: S1083-8791(15)00672-2. doi: 10.1016/j.bbmt.2015.10.002. (Epub ahead of print). Total hemolytic complement activity (CH50) and terminal complement activation (sC5b-9) were observed in order to monitor complement activation and response to therapy. Outcomes for treated patients were compared to those for historical controls from our prospective observational study, consisting of 11 individuals that had shown the same high-risk features but had not been treated with eculizumab. Overall survival between the two groups was compared using the Kaplan-Meier and the Log Rank test starting at TMA diagnosis. Patients with high-risk TMA who received eculizumab therapy had much better survival probability (p=0.002). Figure 5 shows a comparison of the overall survival of patients treated with eculizumab vs. untreated patients with high risk TMA. As shown in Figure 5, after 30 months the patients treated with eculizumab had a survival probability of about 0.6 whereas at the same time point patients that were untreated had a survival probability of approximately 0.1.

## Claims

1. An *in vitro* method for assigning a risk level and severity of thrombotic microangiopathy in a subject comprising:
determining genetic variants in complement-associated genes in a biological sample containing genetic material from a subject, wherein the genetic variants comprise a genetic variant in a CFH gene comprising a change from a cytosine to an adenine at a position corresponding to position 1281 in the cDNA sequence of the CFH gene, and at least one additional genetic variant in an additional complement-associated gene; and
assigning a risk level for the development of thrombotic microangiopathy, and for the likely severity of the thrombotic microangiopathy, based on the genetic variants determined in the biological sample, wherein the risk level corresponds to low risk where no genetic variants are identified, elevated risk where one genetic variant is identified, and high risk where two or more genetic variants are identified.

2. The method of claim 1, wherein the at least one additional genetic variant is selected from Table 2.

3. The method of claim 1 wherein the risk level is determined for a subject prior to the subject being the recipient of a hematopoietic stem cell transplant.

4. The method of claim 1, wherein the biological sample is blood, saliva, skin, or other tissue or fluid.

5. The method of claim 1, wherein determining the genetic variants comprises determining the nucleotide sequence of the complement-associated genes.

6. A kit for the assigning a risk level and severity of thrombotic microangiopathy in a subject comprising:
a set of nucleic acid primers configured to amplify or sequence complement-mediated genes from the subject; wherein the set of nucleic acid primers comprises primers for amplification or sequencing the CD46, THBD, CFH, CFI, CFB, C3, C5, CFD, CFHR1, CFHR3, CFHR4, CFHR5, CD55, and ADAMTS13 genes or for any subset thereof; and
probes configured to bind with a sequence of a genetic variant, wherein the genetic variant is a change from a cytosine to an adenine at a position corresponding to position 1281 in the cDNA sequence of a CFH gene;
wherein the risk level corresponds to low risk where no genetic variants are identified, elevated risk where one genetic variant is identified, and high risk where two or more genetic variants are identified.

7. The kit of claim 6, further comprising probes configured to bind with a sequence of an additional genetic variant selected from Table 2.

8. The kit of claim 6, wherein the probes are labeled with a fluorescent dye, colorimetric dye, radioisotope, or spin-label.

9. The kit of claim 8, wherein the probes are presented on or attached to a solid support.

10. The kit of claim 6, wherein the primers for amplification of said loci incorporate a sequence tag.

11. The kit of claim 10, wherein the primers are presented on or attached to a solid support.

12. The kit of claim 6, further comprising a sample collection device selected from the group consisting of a sample collection container, a sample collection tool, and a sample preservative.

## Patentansprüche

1. *In-vitro*-Verfahren zum Zuweisen eines Risikograds und einer Schwere von thrombotischer Mikroangiopathie in einem Subjekt, das Folgendes umfasst:
Bestimmen genetischer Varianten in komplementassoziierten Genen in einer biologischen Probe, die genetisches Material aus einem Subjekt enthält, wobei die genetischen Varianten eine genetische Variante in einem CFH-Gen, die einen Wechsel von einem Cytosin zu einem Adenin an einer Position, die der Position 1281 in der cDNA-Sequenz des CFH-Gens entspricht, umfasst, und wenigstens eine zusätzliche genetische Variante in einem zusätzlichen komplementassoziierten Gen umfassen; und
Zuweisen eines Risikograds für die Entwicklung von thrombotischer Mikroangiopathie und für die wahrscheinliche Schwere der thrombotischen Mikroangiopathie basierend auf den genetischen Varianten, die in der biologischen Probe bestimmt werden, wobei der Risikograd einem geringen Risiko, bei dem keine genetischen Varianten identifiziert werden, einem erhöhten Risiko, bei dem eine genetische Variante identifiziert wird, und einem hohen Risiko, wenn zwei oder mehr genetische Varianten identifiziert werden, entspricht.

2. Verfahren nach Anspruch 1, wobei die wenigstens eine zusätzliche genetische Variante aus Tabelle 2 ausgewählt ist.

3. Verfahren nach Anspruch 1, wobei der Risikograd für ein Subjekt bestimmt wird, bevor das Subjekt der Empfänger einer blutbildenden Stammzelltransplantation wird.

4. Verfahren nach Anspruch 1, wobei die biologische Probe Blut, Speichel, Haut oder ein anderes Gewebe oder ein anderes Fluid ist.

5. Verfahren nach Anspruch 1, wobei das Bestimmen der genetischen Varianten das Bestimmen der Nukleotidsequenz der komplementassoziierten Gene umfasst.

6. Kit für das Zuweisen eines Risikograds und einer Schwere von thrombotischer Mikroangiopathie in einem Subjekt, der Folgendes umfasst:
einen Satz von Nukleinsäureprimern, der konfiguriert ist, um komplementvermittelte Gene aus dem Subjekt zu amplifizieren oder zu sequenzieren; wobei der Satz von Nukleinsäureprimern Primer für eine Amplifikation oder eine Sequenzierung der Gene CD46, THBD, CFH, CFI, CFB, C3, C5, CFD, CFHR1, CFHR3, CFHR4, CFHR5, CD55 und ADAMTS13 oder für einen beliebigen Teilsatz davon umfasst; und
Sonden, die konfiguriert sind, um an eine Sequenz einer genetischen Variante zu binden, wobei die genetische Variante ein Wechsel von einem Cytosin zu einem Adenin an einer Position ist, die der Position 1281 in der cDNA-Sequenz eines CFH-Gens entspricht;
wobei der Risikograd einem geringen Risiko, bei dem keine genetischen Varianten identifiziert werden, einem erhöhten Risiko, bei dem eine genetische Variante identifiziert wird, und einem hohen Risiko, wenn zwei oder mehr genetische Varianten identifiziert werden, entspricht.

7. Kit nach Anspruch 6, der ferner Sonden umfasst, die konfiguriert sind, um an eine Sequenz einer zusätzlichen genetischen Variante, die aus Tabelle 2 ausgewählt ist, zu binden.

8. Kit nach Anspruch 6, wobei die Sonden mit einem Fluoreszenzfarbstoff, einem kolorimetrischen Farbstoff, einem Radioisotop oder einer Spinmarkierung markiert sind.

9. Kit nach Anspruch 8, wobei die Sonden auf einem festen Träger präsentiert oder an diesem befestigt sind.

10. Kit nach Anspruch 6, wobei die Primer für die Amplifikation der Loci einen Sequenztag beinhalten.

11. Kit nach Anspruch 10, wobei die Primer auf einem festen Träger präsentiert oder an diesem befestigt sind.

12. Kit nach Anspruch 6, der ferner eine Probensammelvorrichtung umfasst, die aus der Gruppe ausgewählt ist, die aus einem Probensammelbehälter, einem Probensammelwerkzeug und einem Probenkonservierungsmittel besteht.

## Revendications

1. Procédé *in vitro* permettant d'attribuer un niveau de risque et une gravité à une microangiopathie thrombotique chez un sujet, comprenant :
la détermination des variantes génétiques dans des gènes associés au complément dans un échantillon biologique contenant du matériel génétique d'un sujet, dans lequel les variantes génétiques comprennent une variante génétique dans un gène CFH comprenant un changement d'une cytosine à une adénine à une position correspondant à la position 1281 dans la séquence d'ADNc du gène CFH et au moins une variante génétique supplémentaire dans un gène associé au complément supplémentaire ; et
l'attribution d'un niveau de risque du développement de la microangiopathie thrombotique et de la gravité probable de la microangiopathie thrombotique, sur la base des variantes génétiques déterminées dans l'échantillon biologique, dans lequel le niveau de risque correspond à un risque faible lorsqu'aucune variante génétique n'est identifiée, à un risque élevé lorsqu'une variante génétique est identifiée, et à un haut risque lorsque deux variantes génétiques ou plus sont identifiées.

2. Procédé selon la revendication 1, dans lequel l'au moins une variante génétique supplémentaire est choisie dans le Tableau 2.

3. Procédé selon la revendication 1, dans lequel le niveau de risque est déterminé pour un sujet avant que le sujet ne soit le receveur d'une greffe de cellules souches hématopoïétiques.

4. Procédé selon la revendication 1, dans lequel l'échantillon biologique est du sang, de la salive, de la peau ou un autre tissu ou fluide.

5. Procédé selon la revendication 1, dans lequel la détermination des variantes génétiques comprend la détermination de la séquence nucléotidique des gènes associés au complément.

6. Kit pour l'attribution d'un niveau de risque et une gravité à une microangiopathie thrombotique chez un sujet, comprenant :
un ensemble d'amorces d'acide nucléique conçues pour amplifier ou séquencer des gènes médiés par le complément chez le sujet ; dans lequel l'ensemble d'amorces d'acide nucléique comprend des amorces pour l'amplification ou le séquençage des gènes CD46, THBD, CFH, CFI, CFB, C3, C5, CFD, CFHR1, CFHR3, CFHR4, CFHR5, CD55 et ADAMTS13 ou pour tout sous-ensemble de ceux-ci ; et
des sondes conçues pour se lier à une séquence d'une variante génétique, la variante génétique étant un changement d'une cytosine à une adénine à une position correspondant à la position 1281 dans la séquence d'ADNc d'un gène CFH ;
dans lequel le niveau de risque correspond à un risque faible lorsqu'aucune variante génétique n'est identifiée, à un risque élevé lorsqu'une variante génétique est identifiée et à un risque élevé lorsque deux variantes génétiques ou plus sont identifiées.

7. Kit selon la revendication 6, comprenant en outre des sondes conçues pour se lier à une séquence d'une variante génétique supplémentaire choisie dans le Tableau 2.

8. Kit selon la revendication 6, dans lequel les sondes sont marquées avec une coloration fluorescente, une coloration colorimétrique, un radio-isotope ou un marqueur spin.

9. Kit selon la revendication 8, dans lequel les sondes sont présentées ou fixées sur un support solide.

10. Kit selon la revendication 6, dans lequel les amorces pour l'amplification desdits locus incorporent un marqueur de séquence.

11. Kit selon la revendication 10, dans lequel les amorces sont présentées ou fixées sur un support solide.

12. Kit selon la revendication 6, comprenant en outre un dispositif de collecte d'échantillon choisi dans le groupe constitué d'un récipient de collecte d'échantillon, d'un outil de collecte d'échantillon et d'un conservateur d'échantillon.
